# EUROPÄISCHE PATENTANMELDUNG

(11) **EP 1 363 129 A1**
(43) Veröffentlichungstag der Anmeldung: **19.11.2003**
(21) Anmeldenummer: 03010635.5
(22) Anmeldetag: 13.05.2003
(51) Int. Cl.: G01N 33/86

(54) **Kontroll-und Kalibrationsmaterial für Blutgerinnungstests**

(30) Priorität: 16.05.2002 DE 10222234
(71) Anmelder: Roche Diagnostics GmbH, 68305 Mannheim (DE); F. HOFFMANN-LA ROCHE AG, 4070 Basel (CH)
(72) Erfinder: Wielinger, Hans, Dr., 69469 Weinheim (DE); Fuellemann, Rainer, Dr., 69469 Weinheim (DE); Abel, Erika, 68167 Mannheim (DE)

(57) **Zusammenfassung**

Die Erfindung betrifft Kontroll- und/oder Kalibrationsmaterialien für Gerinnungsteste, die Plasma und eine Substanz oder ein Substanzgemisch, das die Gerinnungszeit des Plasmas verlängert, enthalten.

## Beschreibung

Die Erfindung betrifft Kontroll- und Kalibrationsmaterialien zur Qualitätskontrolle bzw. Kalibration von Gerinnungstesten, sowie deren Herstellung und Verwendung.

Gerinnungsphysiologische Untersuchungen finden in Krankenhäusern, Arztpraxen, Laboratorien u.a. zur präoperativen Untersuchung, zur Erkennung von Blutungs- und Thromboserisiken, zur Überwachung der Antikoagulantien-Therapie bei thrombosegefährdeten Patienten und bei der Verlaufskontrolle zahlreicher Erkrankungen, z. B. schweren Infektionen, Leberfunktionsstörungen und malignen Erkrankungen, statt. Vermehrt wird die Bestimmung der Prothrombinzeit zum Selfmonitoring der Antikoagulantien-Therapie vom Patienten selbst durchgerührt. Bei all diesen Untersuchungen ist die Qualitätskontrolle ein unabdingbares Muss, um das Testsystem zu überwachen und die Ergebnisse mit größtmöglicher Sicherheit zu erarbeiten.

Die am häufigsten durchgeführten Gerinnungstests sind Globaltests, bei denen gleichzeitig die Funktion mehrerer der am Gerinnungsvorgang beteiligten Faktoren und deren Zusammenwirken in der Gerinnungskaskade bestimmt wird. Als wichtigste Globalteste gelten der sog. Prothrombinzeit Test (abgekürzt PT), auch Quick Test genannt, der sog. aktivierte partielle Thromboplastinzeit Test (aPTT), sowie die Bestimmung der aktivierten Clotting - Time (ACT). Bei all diesen Testen wird die Zeit gemessen, die vom Start des entsprechenden Gerinnungsvorganges mit dem jeweiligen Reagenz bis zum Abschluss des Gerinnungsvorgangs vergeht. Der Gerinnungsvorgang gilt als abgeschlossen, wenn sich ein Gerinnsel gebildet hat. Typischerweise wird der Gerinnungsvorgang dabei über sekundäre, in der Probe beobachtbare Phänomene verfolgt (z. B. über Trübungsmessungen, Messung der Lichtstreuung, der Leitfähigkeit, der Viskosität oder der Aktivität von Schlüsselenzymen der Gerinnungskaskade, wie z. B. Thrombin).

Zur Qualitätskontrolle bzw. Kalibration dieser Verfahren werden Kontroll- bzw. Kalibrationsmaterialien benötigt, mit denen sowohl der Normalbereich (d. h. Proben mit normaler, nicht verlängerter Gerinnungszeit) als auch die Bereiche mit verlängerten Gerinnungszeiten (sogenannter pathologischer Bereich) überwacht bzw. kalibriert werden können.

Als Kontroll- bzw. Kalibrationsmaterialien (im Folgenden der Einfachheit halber auch als Kontrollmaterialien bezeichnet) werden in der Regel lyophilisierte Plasmen, bevorzugt Citratplasmen, verwendet. Diese Plasmen werden meist aus Tierblut gewonnen. Die Lyophilisate werden vor ihrer Verwendung mit destilliertem Wasser oder Pufferlösungen rehydratisiert. Für die Herstellung von Kontrollmaterialien zur Kontrolle des Normalbereichs werden Plasmen ohne spezielle Behandlung verwendet. Zur Herstellung von Kontrollmaterialien für die pathologischen Bereiche (d. h. für Proben mit verlängerter Gerinnungszeit im Vergleich zum Normalbereich) ist es nach dem heutigen Stand der Technik notwendig, ausgehend von Plasmen mit Gerinnungszeiten im Normalbereich über ein relativ kompliziertes Verfahren zu Kontrollmaterialien mit erhöhten Gerinnungszeiten zu kommen. Dazu werden die Plasmen über mit Bariumsulfat gefüllte Säulen chromatographiert. Ein Teil der Gerinnungsfaktoren wird an das Bariumsulfat gebunden, was dazu führt, dass in den so erhaltenen, gerinnungsfaktorabgereicherten Plasmen die Gerinnungszeiten verlängert werden.

Es ist für den Fachmann klar, dass bei den derzeitig gebräuchlichen Verfahren zur Herstellung von Kontrollmaterialien für den pathologischen Bereich nur schwer gut reproduzierbare Ergebnisse erhalten werden und dass vor dem Lyophilisationsschritt die gewünschten Gerinnungszeiten durch nachträgliches Abmischen von gerinnungsfaktorabgereicherten Plasmen mit unbehandelten Plasmen eingestellt werden muss. Dieses Verfahren ist zudem zeit- und materialaufwändig und daher teuer.

Aufgabe der vorliegenden Erfindung war es, ein Verfahren bereitzustellen, mit dessen Hilfe die Herstellung von Kontroll- bzw. Kalibrationsmaterialen für den pathologischen Bereich von Gerinnungstesten, bevorzugt Globaltests, vereinfacht und damit billiger wird, als nach den herkömmlichen Verfahren. Zudem sollten Kontrollmaterialien bereitgestellt werden, die einfacher und reproduzierbarer herzustellen sind als die bislang üblichen Materialien.

Diese Aufgabe wurde durch den Gegenstand der Erfindung, wie er in den unabhängigen Patentansprüchen definiert ist, gelöst. Bevorzugte Ausführungsformen der Erfindung sind Gegenstand der abhängigen Ansprüche.

Es sind Substanzen, wie zum Beispiel Harnstoff (oder Derivate hiervon oder deren Salze) oder Guanidin (oder Derivate hiervon oder deren Salze), bekannt, die die Eigenschaft haben, Proteine durch Einfluss auf deren Sekundär- und/oder Tertiärstruktur zu denaturieren, in dem sie sie beispielsweise partiell entknäueln (siehe hierzu z. B. Lubert Stryer, Biochemie, 1990, Spektrum der Wissenschaft Verlagsgesellschaft mbH, 6900 Heidelberg, Seite 32 - 34). Diese und weitere Verbindungen werden auch in US 5,508,202, Spalte 4, Zeilen 31 bis 51 (auf diese Passage und die darin genannten Substanzen wird hier ausdrücklich und vollumfänglich Bezug genommen) als "Fibrin Precipitations Inhibitoren" beschrieben, die dazu verwendet werden, die Bestimmung der Aktivität des Faktors XIII (im Folgenden kurz: F XIII) zu verbessern. Hierzu wird die Probe, in der die F XIII Aktivität bestimmt werden soll, mit den Fibrin Precipitation Inhibitoren versetzt, eine Lösung von Thrombin und Calcium- Ionen zum Start der letzten Stufe der Gerinnungskaskade dazugegeben, und die Zeit gemessen, die vergeht bis sich das Gerinnsel in der Probe bildet. Zur Bestimmung der F XIII Aktivität in der Probe werden bei dem o. g. Verfahren die Gerinnungszeiten der Patientenplasmen mit der Gerinnungszeit von Normalplasma verglichen.

Überraschenderweise hat sich gezeigt, dass die Herstellung von Kontroll- bzw. Kalibrationsmaterialen für Gerinnungsglobaltests vereinfacht, verbilligt und reproduzierbarer wird, wenn die o.g. Substanzen ("Fibrin Precipitations Inhibitoren") den Plasmen, die als Kontrollmaterialien für den pathologischen Bereich verwendet werden sollen, direkt zugefügt oder in der Lösung mit den lyophilisierten Plasmen rehydratisiert werden. Überraschenderweise tritt in den so gewonnen Kontrollplasmen eine Verlängerung der Gerinnungszeiten ein. Die erfindungsgemäß geeigneten Substanzen sollen im Folgenden auch als "Verlängerungssubstanzen" bezeichnet werden.

Als Verlängerungssubstanzen haben sich erfindungsgemäß alle Verbindungen als geeignet herausgestellt, die die o.g. Eigenschaften haben, bzw. Mischungen derselben. Besonders geeignet sind, dies vor allem aus Kostengründen, Guanidin und Harnstoff, aber auch N-substituierte-Guanidine und N-substituierte-Harnstoffe sowie Salze dieser Substanzklassen, können erfindungsgemäß eingesetzt werden.

Es hat sich gezeigt, dass die Gerinnungszeiten im linearen Verhältnis zur eingesetzten Konzentration dieser Verlängerungssubstanzen verlängert werden können. Aus diesem Grund ist es möglich, die Konzentrationen dieser Substanzen über einen großen Bereich zu wählen, je nach dem, um welchen Betrag die Gerinnungszeiten der Kontrollmaterialien verlängert werden sollen. Aus praktische Überlegungen haben sich Konzentrationen von ca. 0,5 bis ca. 6,0 Gewichtsprozent als besonders günstig erwiesen. Es ist aber auch möglich, den erfindungsgemäßen Effekt mit niedrigeren oder höheren Konzentrationen der Verlängerungssubstanzen zu erzielen.

Die erfindungsgemäßen Kontroll- bzw. Kalibrationsmaterialen werden insbesondere wie folgt hergestellt:
Variante 1:
   Nach dem Poolen der Plasmen (d. h. dem Mischen von Plasmen unterschiedlicher Spender) wird die Verlängerungssubstanz in der für die Verlängerung der Gerinnungszeit erforderlichen Konzentration im gepoolten Plasma gelöst. Das gepoolte Plasma wird anschließend üblicherweise in kleinen Portionen in kleine Gefäße abgefüllt und lyophilisiert.
   Zur Verwendung wird das lyophilisierte Plasma in Wasser oder Pufferlösungen aufgelöst und auf ansonsten identische Weise wie eine Probe vermessen. Nach Ermittlung der entsprechenden Sollwerte kann das so hergestellte Material als Kontrolle oder Kalibrator eingesetzt werden.
Variante 2:
   Es wird lyophilisiertes Plasma, das zur Kontrolle im Normalbereich dient, mit Lösungen der Verlängerungssubstanzen aufgelöst. Die so hergestellte Kontrolle wird wie eine Probe vermessen. Vor dem endgültigen Einsatz als Kontroll- oder Kalibrationsmaterial müssen natürlich noch die für die Kombination Lösung der Verlängerungssubstanz und Normalkontrolle entsprechenden Sollwerte ermittelt werden.
   Die nachfolgenden Beispiele sollen die Erfindung erläutern, ohne jedoch beschränkend zu wirken:

### Beispiel 1

### Herstellung und Verwendung des erfindungsgemäßen Kontroll- bzw. Kalibrationsmaterials für das Produkt CoaguChek® Pro ( Test zur Bestimmung der Prothrombinzeit )von Roche Diagnostics

### Beispiel 1a)

### Wirkung der Verlängerungssubstanz bei Zugabe zum Plasma bei der Herstellung des Kontroll- bzw. Kalibrationsmaterials

Citratplasma, das von Kaninchen gewonnen wurde, wird wie folgt weiter verarbeitet.

Ein Teil des Citratplasmas wird unbehandelt in 3,0 ml Portionen in silikonisierte Gefäße gefüllt und lyophilisiert. Vom Rest des Plasmas werden Ansätze hergestellt, bei denen zum Plasma jeweils 2,0% , 3,5% und 5,0% Harnstoff (jeweils Gew.-%) als Verlängerungssubstanz zugegeben werden. Diese Ansätze werden ebenfalls in 3,0 ml Portionen abgefüllt und lyophilisiert.

Nach dem Rehydratisieren der einzelnen Ansätze mit destilliertem Wasser werden die so gewonnen Proben auf die Testkarten von CoaguChek® Pro von Roche Diagnostics aufgetropft und die Sekunden, die bis zum Eintritt der Gerinnung vergehen, auf dem zugehörigen Messgerät CoaguChek® Pro ermittelt.

Die anschließende Tabelle 1 zeigt die Wirkung des zugegebenen Harnstoffs:

**Tabelle 1:**

| Konzentration Harnstoff (Gew.-%) | Zeit bis zum Eintritt der Gerinnung (s) |
|---|---|
| 0,0 | 17,0 |
| 2,0 | 22,9 |
| 3,5 | 29,6 |
| 5,0 | 41,2 |

### Beispiel 1b)

### Wirkung der Verlängerungssubstanz bei Zugabe zur Rehydratisierungslösung

Kommerziell vertriebenes Kontrollmaterial "Level 1" für das System CoaguChek® ( Test zur Bestimmung der Prothrombinzeit )von Roche Diagnostics wird mit wässrigen Lösungen, die 0,0%, 1,0%, 3,0%, 4,0% und 5,0% Harnstoff (jeweils Gew.-%) enthalten, rehydratisiert. Anschließend werden diese Lösungen mit dem CoaguChek® System (Test plus Messinstrument) gemessen.

Die anschließende Tabelle 2 zeigt die Wirkung des zugegebenen Harnstoffs:

**Tabelle 2:**

| Konzentration Harnstoff (Gew.-%) in der Rehydratisierungslösung | Zeit bis zum Eintritt der Gerinnung (s) |
|---|---|
| 0,0 | 17,0 |
| 1,0 | 19,3 |
| 3,0 | 28,1 |
| 4,0 | 32,2 |
| 5,0 | 37,8 |

### Beispiel 2 :

### Wirkung der erfindungsgemäßen Verlängerungssubstanzen bei Zugabe zu Kontrollplasmen zur Überwachung der Bestimmung der aPTT (aktivierten partiellen Thromboplastinzeit)

### Beispiel 2a)

### Wirkung von N-Methyl-Harnstoff

Das Kontrollplasma "PreciClot I/II" von Roche Diagnostics wird entsprechen der Angabe des Herstellers mit wässrigen Lösungen rehydratisiert, in denen 0%, 2,0%, 3,5% und 5,0% N-Methyl-Harnstoff (jeweils Gew.-%) gelöst sind. Von den so gewonnenen Kontrolllösungen wird mit dem "PTT a Reagenz zur automatischen und manuellen Bestimmung der aktivierten partiellen Thromboplastinzeit" von Roche Diagnostics am Kugelkoagulometer KC 10 A der Firma Amelung (Deutschland) die Gerinnungszeit in Sekunden bestimmt.

Die anschließende Tabelle 3 zeigt die Wirkung des zugegebenen N- Methyl-Harnstoffs:

**Tabelle 3**

| Konzentration N-Methyl-Harnstoff (Gew.-%) in der Rehydratisierungslösung | Zeit bis zum Eintritt der Gerinnung (s) |
|---|---|
| 0 | 31,0 |
| 2,0 | 34,0 |
| 3,5 | 38,6 |
| 5,0 | 44,0 |

### Beispiel 2b)

### Wirkung von Guanidin Hydrochlorid

Der Versuchsaufbau ist der gleiche wie im Beispiel 2a) beschrieben.

In Abänderung werden lediglich folgende Konzentrationen an Guanidin Hydrochlorid zur Rehydratisierungslösung zugewogen (jeweils Gew.-%): 0%, 0,5%, 1,0% und 1,5%.

Die anschließende Tabelle 4 zeigt die Wirkung des zugegebenen Guanidins:

**Tabelle 4**

| Konzentration Guanidin (Gew.-%) in der Rehydratisierungslösung | Zeit bis zum Eintritt der Gerinnung (s) |
|---|---|
| 0 | 31,0 |
| 0,5 | 33,1 |
| 1,0 | 41,9 |
| 1,5 | 51,8 |

### Beispiel 3 :

### Wirkung der erfindungsgemäßen Verlängerungssubstanzen bei Zugabe zu Kontrollplasmen zur Überwachung der Bestimmung der aktivierten Gerinnungszeit (ACT)

### Wirkung von Guanidin

Das lyophilisierte Kontrollplasma "ACT Qualitätskontrolle CoaguChek® Pro System Level 1" von Roche Diagnostics wurde entsprechen der Angabe des Herstellers mit mitgelieferten Rehydratisierungslösungen rehydratisiert. In diese Lösungen wurden 0%, 0,25%, 0,5% und 0,75 % Guanidin Hydrochlorid (jeweils Gew.-%) zugewogen. Anschließend wurde mit den so hergestellten Kontrolllösungen, den ACT Testkassetten CoaguChek®Pro System von Roche Diagnostics und dem entsprechenden Messgerät die Zeit gemessen, die vergeht, bis das Gerät den Eintritt der Gerinnung in den Kontrollplasmapräparationen, die in die Testkassetten pipettiert wurden, anzeigt.

Die folgende Tabelle 5 zeigt die Wirkung des zugegebenen Guanidins:

**Tabelle 5**

| Konzentration Guanidin (Gew.-%) in der Rehydratisierungslösung | Zeit bis zum Eintritt der Gerinnung (s) |
|---|---|
| 0 | 150,1 |
| 0,25 | 175,6 |
| 0,5 | 223,4 |
| 0,75 | 305,6 |

## Patentansprüche

1. Kontroll- und/oder Kalibrationsmaterial für Gerinnungsteste, enthaltend Plasma und
eine Substanz oder ein Substanzgemisch, das die Gerinnungszeit des Plasmas verlängert.

2. Kontroll- und/oder Kalibrationsmaterial gemäß Anspruch 1, **dadurch gekennzeichnet, dass** die Substanz oder das Substanzgemisch, das die Gerinnungszeit des Plasmas verlängert, in der Lage ist, die Sekundär- und/oder Tertiärstruktur von Proteinen, die an der Gerinnungskaskade beteiligt sind, zu verändern.

3. Kontroll- und/oder Kalibrationsmaterial gemäß Anspruch 1 oder 2, **dadurch gekennzeichnet, dass** die Substanz oder das Substanzgemisch, das die Gerinnungszeit des Plasmas verlängert, Proteine entknäuelt.

4. Kontroll- und/oder Kalibrationsmaterial gemäß Anspruch 1, 2 oder 3, **dadurch gekennzeichnet, dass** die Substanz oder das Substanzgemisch, das die Gerinnungszeit des Plasmas verlängert, Harnstoff, oder ein Harnstoffderivat, oder ein Salz von Harnstoff oder ein Salz eines Harnstoffderivats ist.

5. Kontroll- und/oder Kalibrationsmaterial gemäß Anspruch 4, **dadurch gekennzeichnet, dass** das Harnstoffderivat Guanidin oder ein Guanidinderivat ist.

6. Kontroll- und/oder Kalibrationsmaterial gemäß Anspruch 1, 2, 3, 4, oder 5, **dadurch gekennzeichnet, dass** die Substanz oder das Substanzgemisch, das die Gerinnungszeit des Plasmas verlängert, in Mengen von 0,5 bis 6 Gew.-% eingesetzt wird.

7. Verwendung von Substanzen oder Substanzgemischen, die die Gerinnungszeit von Plasma verlängern, als Zusatz zur Herstellung von Kontroll- oder Kalibrationsmaterialien für den pathologischen Bereich für die Gerinnungsmessung.

8. Verwendung gemäß Anspruch 7, **dadurch gekennzeichnet, dass** die Substanz oder das Substanzgemisch, das die Gerinnungszeit des Plasmas verlängert, in der Lage ist, die Sekundär- und/oder Tertiärstruktur von Proteinen, die an der Gerinnungskaskade beteiligt sind, zu verändern.

9. Verwendung gemäß Anspruch 7 oder 8, **dadurch gekennzeichnet, dass** die Substanz oder das Substanzgemisch, das die Gerinnungszeit des Plasmas verlängert, Proteine entknäuelt.

10. Verwendung gemäß Anspruch 7, 8 oder 9, **dadurch gekennzeichnet, dass** die Substanz oder das Substanzgemisch, das die Gerinnungszeit des Plasmas verlängert, Harnstoff, oder ein Harnstoffderivat, oder ein Salz von Harnstoff oder ein Salz eines Harnstoffderivats ist.

11. Verwendung gemäß Anspruch 10, **dadurch gekennzeichnet, dass** das Harnstoffderivat Guanidin oder ein Guanidinderivat ist.

12. Verwendung gemäß Anspruch 7, 8, 9, 10, oder 11, **dadurch gekennzeichnet, dass** die Substanz oder das Substanzgemisch, das die Gerinnungszeit des Plasmas verlängert, in Mengen von 0,5 bis 6 Gew.-% eingesetzt wird.

13. Verwendung eines Kontroll- und/oder Kalibrationsmaterials gemäß Anspruch 1, 2, 3, 4, 5, oder 6 zur Kontrolle oder Kalibration von Gerinnungsmesssystemen.

14. Verfahren zur Herstellung eines Kontroll- und/oder Kalibrationsmaterials gemäß Anspruch 1, 2, 3, 4, 5, oder 6 umfassend die Schritte
Bereitstellen eines Plasmas,
Lösen der Substanz oder des Substanzgemisches, das die Gerinnungszeit des Plasmas verlängert, im Plasma, und gegebenenfalls
Lyophilisieren und Rehydratisieren der Mischung.

15. Verfahren zur Herstellung eines Kontroll- und/oder Kalibrationsmaterials gemäß Anspruch 1, 2, 3, 4, 5, oder 6 umfassend die Schritte
Bereitstellen eines lyophilisierten Plasmas, und
Rehydratisieren des Plasmas mit einer Lösung der Substanz oder des Substanzgemisches, das die Gerinnungszeit des Plasmas verlängert.

16. Kit zur Kontrolle oder Kalibrierung von Gerinnungsmesssystemen umfassend lyophilisiertes Plasma und
eine Substanz oder ein Substanzgemisch, das die Gerinnungszeit des Plasmas verlängert.
